# EUROPEAN PATENT SPECIFICATION

(11) **EP 3 103 404 B1**
(45) Date of publication and mention of the grant of the patent: **01.09.2021**
(21) Application number: 16177750.3
(22) Date of filing: 03.10.2007
(51) Int. Cl.: A61B 17/22, A61B 17/3207, A61M 1/00

(54) **INTERVENTIONAL CATHETERS**
INTERVENTIONSKATHETER
CATHÉTERS CHIRURGICAUX

(30) Priority: 04.10.2006 US 828209 P; 09.03.2007 US 894173 P
(43) Date of publication of application: 14.12.2016
(62) Divisional of application: 07843776.1
(73) Proprietor: Boston Scientific Limited, Hamilton HM11 (BM)
(72) Inventor: TORRANCE, Casey, Snohomish, WA 98290 (US); WALSH, Kate, Kirkland, WA 98033 (US); SALSTROM, Jarod, Renton, WA 98056 (US); YOUMANS, Scott, Bothell, WA 98011 (US); AUTH, David, Kirkland, WA 98033 (US); WULFMAN, Edward I., Woodinville, WA 98072 (US)
(74) Representative: Vossius & Partner Patentanwälte Rechtsanwälte mbB

(56) References cited:
- WO-A-95/21576
- WO-A-2004/080507
- US-A1- 2005 085 769
- US-B1- 6 503 261
- US-B1- 6 565 588

## Description

### Field of the Invention

The present invention relates to systems for removing material, such as obstructions and partial obstructions, from an internal lumen or cavity of a mammalian subject, such as a blood vessel. More particularly, the present invention relates to interventional catheters having operating heads incorporating structures for removing obstructions and partial obstructions from a lumen or cavity, and aspiration/infusion and/or mastication systems for removal of material cleared from the target site.

### Background of the Invention

Removal of disease such as atherosclerotic plaque, thrombus and other types of obstructions and partial obstructions from internal body lumens or cavities using advanceable, rotating operating heads having cutter assemblies or abrasive materials is a well-established interventional technique. Numerous interventional catheters have been conceived and developed. Most of these systems require placement of a guiding catheter and guide wire prior to introduction of the interventional catheter and placement of the interventional catheter at the target operating site. Many of these prior art systems incorporate mechanical aspiration systems to remove the ablated material from the site and some systems incorporate, or are used in conjunction with, other mechanisms such as distal filters for preventing removed material from circulating in the blood stream

Despite the many and varied approaches to the material removal systems, many challenges remain in providing systems for removing material from a lumen, such as a blood vessel, safely and reliably and without causing complications. The safety and reliability of the system is manifestly critical. Recovery of debris generated during a material removal operation, or maceration of the debris to a particle size that will not produce blood vessel damage or embolic events, is essential. The flexibility and size of an interventional catheter are also important features. The system must be small enough and flexible enough to navigate through sometimes tortuous internal structures and passageways, such as blood vessels, for placement at the target interventional site. The interventional catheter must also have sufficient integrity and a combination of stiffness and flexibility to operate reliably at high rotational rates while allowing for aspiration and/or infusion of fluids to the site.

In interventional catheters that employ a "cutting head," any cutter structures must be benign during navigation of the operating head to and from the interventional target site, yet effectively remove material during the operation. In addition, cutter structures must effectively remove disease or undesired material without damaging delicate neighboring tissue, such as blood vessel walls or other healthy tissue, which often surrounds and may be attached to the undesired material. Thus, it is important for cutter structures of interventional catheters to accurately and reliably differentiate between the diseased or undesired material and healthy tissue.

Differential cutting blades exert high shear forces against relatively hard substrates to cut or ablate relatively hard, inelastic, material. Softer, elastic structures, such as healthy tissue, blood vessel walls and the like, are deformed rather than cut by differential cutting blades, thereby reducing the shear forces and protecting elastic structures from damage. Less elastic material does not deform when contacted by a differential cutting blade, and shear stresses are consequently exerted on less elastic material to cut or scrape and ablate the material without damaging elastic tissue in proximity. In this manner, fragments of diseased, undesirable material are removed by differential cutting blades, while the more elastic, healthy tissue remains undamaged.

U.S. Patent 4,445,509 describes differential cutting in the context of an atherectomy device. This patent describes a cutter assembly having a plurality of cutting flutes, each cutting flute having a blade surface operating according to the principle of differential cutting. Aspiration ports are provided in the body of the cutter assembly for collection and removal of particulates and liquids from the site of the intervention. U.S. Patent Publication 2004/0006358 A1 also discloses differential cutting surfaces forming an acute angle of attack with respect to the occlusive material. Aspiration ports are provided between the cutting surfaces.

Some interventional catheters use diamond grit on a cutting surface in an effort to provide highly divided, small particle size debris. Diamond grit particles, however, do not operate as differential cutters except in their smallest embodiment because, depending on their orientation on the cutting surface, their exposed surfaces form random cant angles producing different cutting characteristics at different points of contact with tissue. Relatively coarse diamond grit can act as a differential cutter because of the ratios of diamond size and population to tissue flexibility, but typically is more likely to damage elastic, healthy tissue such as blood vessel walls. Relatively fine diamond grit has slow material removal rates, requiring the use of higher rotational speeds. The use of grit or abrasive particles or surfaces is, however, beneficial in many applications.

The extent and consistency of the disease or undesired material forming an obstruction are frequently not well characterized prior to intervention. Thus, although interventional catheters and cutter assemblies having different sizes and material removal properties may be provided, and may even be interchangeable on a material removal system, it is difficult to ascertain which combination of features will be most effective in any particular intervention prior to insertion of the device. Various quick-connect systems have been developed to permit removal and installation of multiple operating catheters during a single surgical intervention. This is not ideal, since interchange, withdrawal and insertion of multiple interventional catheters is time consuming, may result in increased blood loss and increases the risk to the patient.

Providing access to multiple cutter assemblies having different sizes and different material removal properties on a single interventional operating catheter is highly desirable. Interventional catheters having cutter or material removal assemblies that can be operated to vary the size of the cutting profile at the material removal site are known. Cutter assemblies comprising a distal cutter assembly having fixed blades and a proximal cutter assembly having pivoting blades are described, for example, in U.S. Patents 6,565,588 and 6,818,001.

Alternative material removal systems may incorporate an Archimedes screw-type mechanism at a distal end of an interventional catheter, in which material is caught between the threads of the screw and withdrawn from the site using mechanical rotational motion. Material removal systems may also incorporate a plaque excision device having a blade that traverses and exits a window at a distal end of an interventional catheter to scrape plaque from a vessel wall and collect it in an internal collection space provided in the distal end of the interventional catheter.

In any of these material removal systems, removal of debris generated at the site of the intervention is critical to prevent distal embolization of the debris. Several prior art interventional catheters provide for aspiration of liquids and/or debris from the material removal site. Aspirating thrombectomy catheters employ a catheter having a vacuum system to draw thrombus into the catheter and remove it from the site. Many interventional catheters incorporate, or are used with, a distal filter mechanism that traps debris before it can be carried away in the bloodstream. Numerous interventional catheters also provide infusion of a liquid to the site of the intervention. Infused liquids may also assist in the material removal process, or may be provided as diagnostic or therapeutic materials prior to, during or following an intervention.

Although interventional catheters are used frequently, limitations in the flexibility, reliability and versatility, together with capability of use and performance of existing systems limit the types of disease conditions that can be effectively treated. There thus remains a need for improved interventional catheter assemblies and control systems.

US 6 565 588 B1 discloses an intralumenal material removal system using an advanceable, rotatable and expandable cutter assembly. The intralumenal material removal system includes a cutter assembly positionable in the lumen of a mammalian subject. The cutter assembly comprises a distal cutting head and an adjustable cutting assembly that is axially advanceable by translating the drive shaft and rotatable by rotating the drive shaft. The adjustable cutting assembly is adjustable between a smaller diameter condition and a larger diameter condition. The cutter may thus be introduced to and withdrawn from the material removal site in a retracted, smaller diameter condition that facilitates translation and navigation of the device through various lumens. The adjustable cutting assembly may be selectively expanded at the material removal site to facilitate cutting, removal and aspiration of the occlusive material.

US 6 503 261 B1 discloses an atherectomy burr that includes a number of cutting blades having a less aggressive cutting action when rotated in a first direction and a more aggressive cutting action when rotated in a second direction.

US 2005/0085769 A1 discloses fluid exchange systems for irrigation and aspiration that comprise devices to produce both controlled and localized flow with defined volume exchange ratios for fluid management.

### Summary of Invention

An interventional catheter assembly as recited in claim 1 is provided. The dependent claims define embodiments. No surgical methods form part of the invention.

The present invention provides interventional catheters that may be employed to rapidly and effectively remove unwanted material from body lumens or cavities. Interventional catheters and control systems disclosed herein may be adapted for use within a variety of body lumens or cavities such as blood vessels and vascular cavities, gastrointestinal cavities, lumens or cavities in the urinary system and in male and female reproductive organs, and other fluid cavities such as pulmonary lumens and gas exchange cavities, nasal and sinus cavities and the like. The lumen or cavity may form a generally tubular structure, such as a blood vessel, a ureter, a fallopian tube, a nasal passageway, and other tubular passageways. For example, systems of the present invention may be used for removing undesired material from native blood vessels such as native coronary, renal, cranial, peripheral and other blood vessels, artificial or grafted vessels such as saphenous vein grafts, and the like. The lumen may have implanted devices such as stents in place. The lumen or cavity may be within, or in proximity to, an organ such as a kidney, gall bladder, lung or the like, or the body cavity may form part of another system, such as a lymph node, spinal canal, or the like. Interventional catheters are generally used to remove unwanted material from a target site in body lumens or cavities of mammalian subjects, particularly human patients.

The undesired material that is removed using interventional catheter assemblies and control systems disclosed herein may be disease material such as atherosclerotic plaque, calcified plaque, thrombus, or other types of deposits, gallstones, a valve or portion thereof, and the like. In certain embodiments, the interventional catheter assemblies disclosed herein are employed in the treatment of cardiovascular or peripheral artery disease (PAD) to remove disease material from blood vessels, including peripheral blood vessels.

The present interventional catheter assembly includes a catheter system that is at least partially inserted and navigated within a patient's body while an operator controls the system externally of the patient's body. A control module housing aspiration and/or infusion systems, providing power to a downstream interventional catheter controller, and providing various control and display features may also be provided. The interventional catheters disclosed herein incorporate a material removal component, referred to herein as an "operating head," which is generally positioned at or near the distal end of the interventional catheter system. As used herein, "proximal" refers to a direction toward the system controls and the operator along the path of the catheter system, and "distal" refers to the direction away from the system controls and the operator along the path of the catheter system toward or beyond a terminal end of the operating head.

Fluidic communication between the operating head and externally positioned components of the interventional catheter system is generally provided by one or more sealed passageways of the catheter system. Other types of communication systems or pathways may also be provided for delivery of power, for rotationally driving and translating the operating head, for implementing various control features, and the like. The operating head may be driven, or controlled, using electrical systems, radio frequency and other remote control systems, mechanical systems, magnetic systems and other systems or modalities suitable for remote operation of an operating head. The operating head may also incorporate features providing additional functionalities such as ultrasound guidance, various types of imaging features, and the like. The system components described below are described as exemplary components and are not intended to limit the scope of the invention.

The interventional catheter system may be used in conjunction with a flexible guidewire that is navigated through internal pathways, such as blood vessels, to a target material removal site. For partial obstructions, the guidewire is generally placed across the lesion and the operating head of the interventional catheter is advanced on the guidewire to the target site and then operated into and through the lesion. When a lumen is totally obstructed and a guidewire cannot penetrate the obstruction without causing harm to nearby tissue or risking embolization, the operating head may be advanced beyond the distal tip of the guidewire and into and through the obstruction, or the operating head and guidewire may be advanced in tandem. Other methods that may be employed for guiding and steering the operating head include, but are not limited to, radio frequency systems, stereotactic systems, magnetic systems, remote control systems, and the like. The interventional catheters disclosed herein may be adapted for use with any of these steering systems.

The operating head is rotatable, incorporates cutter elements, and is operably connected to a rotatable and axially translatable drive shaft, drive system and control systems. In preferred embodiments, the operating head comprises at least one blade having a cutting surface that operates according to the principles of differential cutting. Although the "cutting" surfaces or blades of an interventional catheter may be sharp and may actually "cut" material at the target site, the term "cut" or "cutting" or "cutter" or "blade(s)," as used herein, refers to cutting, scraping, abrading, ablating, macerating and otherwise breaking down undesired material into particles or smaller, removable, units of material. Cutter assemblies disclosed herein generally comprise a plurality of differential cutting blades and may incorporate fixed and/or adjustable blades as disclosed, for example, in U.S. Patents 6,565,588 and 6,818,001. Differential cutting blades are also disclosed, for example, in U.S. Patent Publication 2004/0006358 A1. In some embodiments, the operating head may comprise an abrasive surface or an abrasive material provided on a surface of a rotational element. Rotational elements incorporating abrasives are well known in the art. In an alternative embodiment, the operating head may comprise another type of ablation device, such as a plaque excision device, a laser ablation or high frequency ultrasound ablation device, or a radio frequency or heat-producing or electrical device that operates to remove unwanted material from body lumens or cavities and generally does not rotate during operation. These ablation devices are also well known in the art.

In one embodiment, interventional catheters have an operating head incorporating a cutter assembly having a plurality of radially arranged, fixed blades, each blade providing a differential cutting surface at a leading edge. The term "leading" edge or surface, as used herein, indicates the edge or surface that contacts material during rotation of the operating head in a direction to achieve removal of material by contact with the edge or surface, and the term "trailing" edge or surface, as used herein, indicates the edge or surface generally "behind" or on an opposite side of the leading edge. In one embodiment, a fixed blade cutter assembly comprises a plurality of raised cutting surfaces having depressions between adjacent cutting surfaces, with both the blade and depression structures terminating in a generally smooth distal collar.

Cutter assemblies may optionally incorporate openings or ports providing access to an internal cavity communicating, for example, with a sealed lumen of the catheter for aspiration and/or infusion of fluids. Ports may be provided in a fixed blade or adjustable blade cutter assembly, or both. In a fixed blade cutter assembly, ports may be located between all or a portion of the cutting surfaces and are preferably provided in a radially symmetrical arrangement in a generally proximal portion of the cutter assembly. In alternative cutter assemblies, there are no ports or openings providing access to the interior of the cutter assembly.

The cutter assembly may comprise one or more cutters or cutting surfaces and one or more distinct types of cutter elements. For example, a dual cutter configuration incorporates a fixed diameter cutter and an adjustable diameter cutter in combination. Dual cutter assembly configurations are described in the prior art publications incorporated herein by reference and may present two different material removal profiles in two different operational modes. In one mode, the cutter assembly is rotated and advanced to remove occlusive material in an initial "pilot pass" in which the fixed diameter cutter is the primary cutter, and the adjustable diameter cutter is in a smaller diameter condition. Following one or more pilot passes, the adjustable diameter cutter may be adjusted to a larger diameter condition in a second mode of operation, and the dual cutter assembly may be advanced so that the adjustable diameter cutter, in its expanded condition, operates as the primary cutter and clears an even larger volume of occlusive material. Following this material removal operation, the adjustable diameter cutter may be adjusted to a smaller diameter condition and the dual cutter assembly may be withdrawn from the site. This method, using the material removal system of the present invention, obviates the need for the operator to remove and replace, or interchange, cutter assemblies during a material removal operation to provide cutters having different diameters and material removal capabilities.

According to one embodiment, fixed blade cutter assemblies of the present invention incorporate differential cutting surfaces having a leading edge cant angle of less than 90°, and preferably incorporate differential cutting surfaces having a leading edge cant angle of less than 80°. The trailing edge cant angle may be different from the leading edge cant angle, and is generally less than the leading edge cant angle. Fixed blade cutter assemblies may incorporate differential cutting surfaces having generally planar leading and trailing faces. In one embodiment, the generally planar differential cutting surfaces are continuous and generally smooth, and they do not provide any communication with internal spaces of the cutter assembly. In general, any number of blades, generally from about three to about twelve, may be provided in a radially symmetrical arrangement. In one illustrative embodiment, ten fixed cutter blades are provided in a cutter assembly having multiple ports providing access to an internal cavity. In another illustrative embodiment, four fixed cutter blades having smooth, continuous surfaces with no openings are provided.

Interventional catheters of the present invention incorporating a fixed blade cutter assembly may additionally comprise cutter blades presenting differential cutting surfaces having a different configuration, profile and/or cant angle. Additional cutter surfaces may be provided proximally to the fixed blade cutter assembly, for example, as adjustable (e.g., pivoting) cutting blades, as described in the patent publications incorporated herein by reference. Pivoting cutting blades may be mounted for limited rotation on a rotating structure with or without ports that communicate with an internal space for aspiration and/or infusion. In general, any number of blades, generally from about three to about twelve, may be provided in a radially symmetrical arrangement. According to one embodiment, adjustable blade cutter assemblies of the present invention may incorporate differential cutting surfaces having a leading edge cant angle of less than 100° and greater than 80°.

Cutter assemblies comprising an operating head having a combination of cutter blades and another material removal modality are also contemplated. In some embodiments, fixed or adjustable cutting blades may be provided in an operating head in combination with a non-cutting material removal system, such as a laser-based device, a high frequency ultrasound device, or a heating and/or electrically ablative device. The fixed or adjustable cutting blades may be positioned proximal to or distal to the non-cutting material removal system.

Cutter assemblies incorporating cutter surfaces having a stepped configuration are disclosed herein and are preferred for many applications. Stepped blades have a raised cutting surface or blade positioned adjacent a shoulder portion that is generally atraumatic to tissue. This blade configuration essentially limits the depth of tissue cut during rotation of the blade to the depth of the raised cutting surface. Both fixed and adjustable cutting blades may be provided with stepped cutting surfaces. More aggressive or less aggressive blades may be designed and provided, depending on the dimensions of the raised cutting surface, the cant angle of the cutting edge of the raised cutting surface, and the relative dimensions of the raised cutting surface, the width of the shoulder portion and other blade surface geometries. Suitable stepped cutter blade geometries may incorporate a leading edge cant angle of greater than 70°, more preferably greater then 80°, and in some embodiments between about 80° and 110°. These blades present a more aggressive leading cutting edge than blades having a lower cant angle, but they demonstrate a reduced and generally more benign cutting profile because the effective cutting edge is limited by the configuration of the cutting edge and the presence of the generally atraumatic shoulder portion.

In applications employing rotational operating heads, the drive shaft that conveys rotation and torque from a drive system to the operating head is small enough and flexible enough to be navigated through small and tortuous passageways during navigation of the operating head to the target removal site. It also has sufficient mechanical integrity to transfer high rotational and torque loads, and operate in a high vacuum, or aspirate withdrawal, environment. Multi-filar helical coils are used as drive shafts in many types of interventional catheters having a rotatable operating head.

The drive shaft is carried in a flexible catheter structure and mounted, directly or indirectly, to the operating head to rotate the operating head. The rotational operating head and drive shaft may be directly or indirectly connected to the flexible catheter structure by means of a bearing near the distal end, such that the catheter remains stationary during operation of the operating head, while the operating head is rotated by the drive shaft. Alternatively, the operating head and drive shaft may be independent of the catheter at its distal end, such that the operating head is rotatable and axially translatable independent of the catheter assembly.

Interventional catheters disclosed herein preferably incorporate an aspiration system for removal of debris from the intervention site by means of aspiration through one or more aspiration ports. Aspiration systems suitable for use in interventional catheters of the present invention are described, for example, in the patents incorporated herein by reference and in U.S. Patent Publication 2004/0220519 A1. Debris generated during a material removal operation is entrained in fluids (e.g. blood), and the aspirate fluid containing debris is removed through the material removal port(s) and withdrawn through a sealed lumen of the interventional catheter. The sealed lumen is connectable to a vacuum source and aspirate collection system.

In one embodiment of interventional catheters of the present invention, at least one aspiration port having a generally large opening is provided in proximity to the operating head (e.g., the cutter or plaque excision assembly). The aspiration port may be provided directly in a catheter structure, or it may be provided in a rigid shell structure mounted directly or indirectly to a distal portion of the catheter. Debris generated during a material removal operation is entrained in fluids (e.g. blood and infusate), and the aspirate fluid containing debris is removed by aspiration through the material removal port(s) and withdrawn through a sealed lumen of the interventional catheter. The sealed lumen is connectable to an aspirate conduit and aspirate collection system. The least one aspiration port is positioned proximal to the operating head. In some embodiments, at least one large aspiration port is positioned proximal to the operating head. Generally smaller material aspiration ports may additionally be disposed on one or more surfaces of the cutter assembly itself.

The interventional catheter may additionally incorporate a masticator assembly positioned for rotation inside the aspiration port to facilitate removal and breaking down of debris withdrawn from the site of intervention through the port. The rotatable masticator assembly may comprise a central core structure having one or more projecting bars, extending generally along the length of the central core. The central core structure is rotated during operation of the operating head. When the operating head comprises a rotating structure, the masticator assembly may be directly or indirectly connected to the same drive system that rotates the operating head.

The outer surfaces of the projecting bars provided on the masticator central core structure are generally curved and, in some embodiments, generally match the inner surface of a shell structure in which the aspiration port is provided. The projecting bars may have a tapered structure, with a narrower profile toward a proximal end of the masticator assembly and a wider profile toward a distal end of the masticator assembly. The bars may also be configured so that their side walls have different dimensions and different undercut angles along the length of the bars. In general, the side walls are higher and have a smaller undercut angle toward a proximal end of the masticator assembly and are lower and have a larger undercut angle toward a distal end of the masticator assembly. As particulate material is drawn into the aspiration port by aspiration forces, the rotating macerator assembly facilitates further breakdown of the material and assists in moving the particulate material proximally and through the aspiration conduit for downstream collection and disposal.

Liquid infusion may be provided in, or in proximity to, the operating head. Infusion of liquids may be used to provide additional liquid volume for removal of debris, or to deliver lubricating fluids, diagnostic or treatment agents, contrast agents and the like. Infusion of fluids such as saline in proximity to the target material removal area may be desirable because it tends to reduce the viscosity of the materials being removed, thus facilitating removal through relatively small diameter lumens. Infusion of liquids also desirably tends to reduce the volume of blood removed during a material removal operation, thereby reducing blood loss and allowing longer procedures if necessary. In addition, infusion of liquids reduces vessel collapse and keeps the vessel wall in tension, thereby improving the effectiveness of cutting and reducing damage to the vessel wall. Liquid infusion may also reduce guidewire friction in embodiments where guidewires are employed. Liquid infusion may be provided distal or proximal to the operating head, and/or may be provided through the operating head.

Many different types of infusion systems are known and may be used in interventional catheters of the present invention. In one embodiment, multiple infusion ports are arranged to provide a substantially even distribution of infusate around the circumference of the device, while in alternative embodiments, one or more infusion ports may be provided to distribute infusate in a directed manner at one or more locations around the circumference of the device. A plurality of fluid infusion ports are provided in an outer sheath mounted over a distal catheter portion and positioned proximal to the operating head in one embodiment. Infusion may additionally be provided through ports in the operating head.

In general, interventional catheters of the present invention operate to provide a volume ratio of infusate to aspirate of greater than about 1:1. For example, the volume ratio of infusate to aspirate may be greater than 1.5:1 and is less than about 2.5:1. Infusion and aspiration rates may also be controlled within desired ranges, and several monitoring and control features may be provided. In one embodiment, for example, a bubble detection mechanism is provided to detect a bubble in the infusion conduit and inactivate the infusion pump and/or power to the operating head upon detection of a bubble. In another embodiment, the infusion and aspiration systems are activated automatically upon activation of the operating head, or after a delay period following activation of the operating head. In yet another embodiment, the infusion and aspiration systems are may also be inactivated automatically upon inactivation of the operating head, or after a delay period following inactivation of the operating head.

### Brief Description of the Drawings

Fig. 1 is a schematic diagram of an interventional catheter assembly having aspiration and infusion systems and comprising an operating head mounted at or near a distal end of a catheter system, a controller and a console unit.
Fig. 2A is an enlarged perspective view of one embodiment of a fixed blade distal cutter assembly.
Fig. 2B is a cross-sectional view, illustrating the leading edge cutter surface cant angles for the differential cutter blades of the cutter assembly of Fig. 2A.
Fig. 3 is an enlarged perspective view of a dual cutter assembly having a distal fixed blade assembly and a proximal adjustable blade assembly.
Fig. 4 is an enlarged perspective view of the fixed blade cutter assembly illustrated in Fig. 3.
Fig. 5A is an enlarged perspective view of a stepped cutter blade.
Fig. 5B is an enlarged cross-sectional view of the stepped blade of Fig. 5A taken through line 5B-5B.
Fig. 6 is an enlarged schematic end view of a cutter assembly incorporating a distal fixed blade cutter assembly as shown in Fig. 4 and an adjustable blade cutter assembly employing stepped cutter blades as illustrated in Figs. 5A and 5B.
Fig. 7 is an enlarged perspective view of another stepped blade configuration.
Fig. 8 illustrates an enlarged perspective view of an interventional catheter of the present invention having a distal operating head with ports for aspiration or infusion and a proximal tubular structure having a relatively large port for aspiration.
Fig. 9 illustrates an enlarged perspective view of an interventional catheter of the present invention having a distal operating head, a proximal aspiration port and liquid infusion ports located proximally to the aspiration port.
Fig. 10 illustrates an enlarged cross-sectional view of an interventional catheter having a distal operating head and a proximal aspiration port with an internal rotating member.
Fig. 11 illustrates an enlarged view of an aspiration window and internal rotating macerator element.
Fig. 12 illustrates an enlarged perspective view of the internal rotating macerator element.
Fig. 13 illustrates an enlarged cross-sectional view of the aspiration assembly of Fig. 5 taken through line 7-7.
Fig. 14 illustrates an enlarged cross-sectional view of the aspiration assembly of Fig. 5 taken through line 8-8.
Fig. 15 illustrates an enlarged cross-sectional view of the aspiration assembly of Fig. 5 taken through line 9-9.

### Detailed Description

Certain preferred embodiments are described herein with reference to a material removal device having a rotational cutting head. It will be appreciated that this device embodiment is being described as illustrative and that the features disclosed herein are applicable to interventional catheters having different types of operating heads.

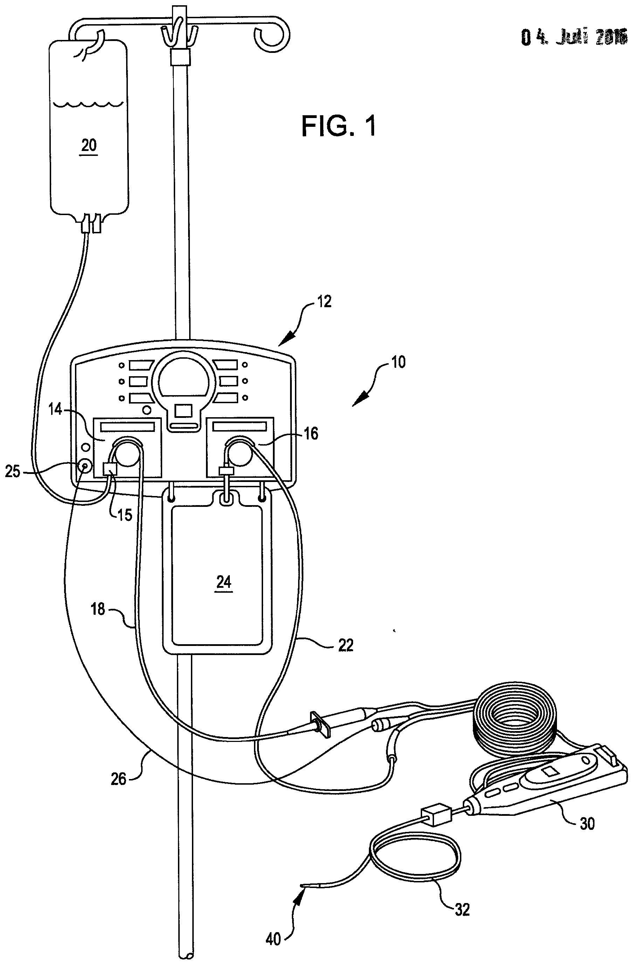

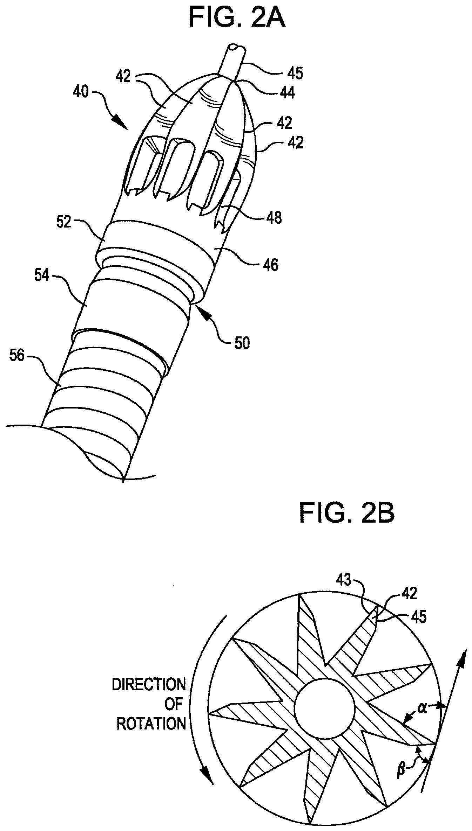

Fig. 1 illustrates an exemplary embodiment of an interventional catheter assembly 10 comprising console unit 12, controller 30, and catheter system 32 having a rotatable operating head 40 located at or in proximity to the distal end of the catheter system and incorporating one or more cutter assemblies. Controller 30 may be used to manipulate (e.g. advance and/or rotate) the catheter system 32 and operating head 40, or alternative controls may be provided. The configuration of the operating head and cutter assemblies will be described below with reference to Figs. 2 -7.

Console unit 12 may incorporate an infusion pump 14 and/or an aspiration pump 16. During operation of the interventional catheter, an infusate conduit 18 draws fluid from an infusate reservoir 20 and operably contacts the infusion pump 14 to provide fluid through an infusion lumen in catheter system 32 to one or more infusion ports provided in proximity to the operating head. Similarly, but in reverse, fluids with entrained particulates may be withdrawn from the site of intervention through an aspiration lumen in catheter system 32 and conveyed to aspiration conduit 22, which is in operable contact with the aspiration pump 16, and communicates with the aspirate collection vessel 24. Console unit 12 may also provide a power source for operating the operating head and system components, or it may be in communication with an external power source. In the illustrated embodiment, console unit 12 provides power to the interventional catheter assembly and controller 30 by means of a device power port 25 and power cord 26.

Various microprocessor, electronic components, software and firmware components may be provided within or in communication with the console unit for controlling operation of the interventional catheter as described herein. Software may be provided in a machine-readable medium storing executable code and/or other data to provide one or a combination of mechanisms to process user-specific data. Alternatively, various systems and components may be controlled using hardware or firmware implementations. Data storage and processing systems may also be provided in console unit 12.

One function of console unit 12 is to provide feedback of system and/or environmental conditions or operating parameters. The console unit may output operational information concerning operating conditions and feedback from the material removal site to the operator. According to one embodiment, console unit 12 provides continuously updated output to an operator of operating parameters such as operating head rotation rate, which may include the actual run speed as well as the desired speed; operating head advance rate; aspiration rate and/or volume; infusion rate and/or volume; elapsed run-time; and the like.

Certain automated and selectable control features may be implemented in console unit 12. Preset routines or programs involving various operating parameters may be preselected, stored and selectable by an operator, for example. Thus, according to one embodiment, the disclosed material removal system implements control features based on an operator's input of specified parameters. Specified parameters may include, for example: lesion length, lesion type and character, such as calcified, fibrotic, lipid/fatty and the like; historical factors, such as restenosis; rate of blood flow; volume of blood flow; percentage of restriction; lumen type and/or location; lumen diameter; desired rotation rate and/or rotation profile for the cutter assembly; desired advance rate and/or advance profile for the cutter assembly; desired aspiration rate and/or profile; desired infusion rate and/or profile; and the like. Based on the specified parameters input by the operator, the control unit may calculate and implement automated operating conditions, such as: cutter assembly rotation rate and profile; cutter assembly advance rate and profile; aspiration rate and profile; infusion rate and profile; cutter assembly size; and the like. Various system operating parameters, operating conditions, patient conditions, and the like may also be recorded and stored during interventions to preserve a record of the patient and intervention operational parameters.

High efficiency aspiration is important in the interventional catheter systems disclosed herein. In certain embodiments, fluid and associated particulates are aspirated from the intervention site at rates of at least 15 ml/min of operating head run time and in many embodiments, fluid and associated particulates are aspirated from the intervention site at rates of at least 15 ml/min of operating head run time and in many embodiments, fluid and associated particulates are aspirated at rates of at least 25 ml/min of operating head run-time. In exemplary interventional catheter systems, the aspiration site may be more than a meter away from the controller 30 through an aspirate removal passageway located within the catheter system 32 and having a diameter of less than 0.10 inch, for example between about 1.27 to 1.778 mm (about 0.050 to 0.070 inch). The distance that the aspirate travels between controller 30 and console unit 12 may be from about 0.5 meter to several meters, through an aspirate conduit that is between about 3.175 to about 25.4 mm (about 0.125 inch to about 1.0 inch) in diameter. The blood and debris being aspirated are relatively viscous fluids, and achieving a relatively constant and high level of aspiration under these conditions is essential.

In one embodiment, aspiration pump 16 comprises a multi-lobed roller pump. The rotation rates of multiple rollers, or of a multi-lobed rotating structure, may be variable or selectable to control the aspiration rate and volume. Roller pumps permit fluid to flow in a conduit through the rollers of the pump at atmospheric pressure, and thus reduce or prevent the formation of bubbles and foam in the liquid being evacuated. Because the aspirate is at atmospheric pressure when it exits the roller pump, a simplified, atmospheric pressure collection vessel may be used rather than an evacuated collection vessel. A simple bag or another collection vessel, such as those used for collection of blood, may be used. For example, a collection bag 24 and a sealed aspiration conduit may be provided as part of a sterile disposable interventional catheter kit. A distal end of the aspiration conduit may be pre-mounted on and sealed to the controller 30. A proximal portion of the aspiration conduit is mounted on the aspiration pump prior to operation of the interventional catheter and the aspirate collection bag is mounted to or in proximity to the control module.

Infusion pump 14 may also comprise a multi-lobed roller pump employing variable or selectable rotation rates to control the infusion rate and volume. A simple bag or another infusate reservoir, such as those used for intravenous infusions, may be used to supply the infusate. For example, an infusate reservoir 20 having a sealed conduit that is mounted in the infusion pump 16 during operation of the interventional catheter may be provided. In this embodiment, the sealed infusate conduit may be provided as part of the sterile disposable interventional catheter system and a distal end of the infusate conduit may be pre-mounted on and scaled to the controller 30. A proximal portion of the infusate conduit may be connected to an infusate reservoir, such as a saline bag, and mounted in proximity to the infusion pump prior to operation. A bubble detector 15 may be provided in association with console unit 12 and infusate conduit 18 to detect the presence of gas bubbles in the infusate. A control feature that automatically disables the infusion pump and/or power to the operating head may be activated upon detection of a fault (e.g. a bubble) in the infusate conduit.

Console unit 12 may also have control switches for activating and shutting down the aspiration pump and system, and for activating and shutting down the infusion pump and system. These control features may be provided as simple on/off switches. Alternatively, systems providing different levels or rates of aspiration and/or infusion that are selectable by an operator may be provided. In addition, console unit 12 may be provided with a timing mechanism that determines, and displays, the elapsed time of operation of the operating head and/or the aspiration and infusion systems. The volumes of aspirate withdrawn and the volume of infusate introduced may also be detected and displayed by console unit 12. Detection systems for monitoring the levels of aspirate and infusate in the respective reservoirs may be incorporated and alarms indicating an overfill condition for the aspirate collection system or a low supply condition for the infusate reservoir may be provided. Back-up aspirate collection and infusate supply systems may also be provided.

In one embodiment, console unit 12, together with aspiration pump 16 and infusion pump 14 and the associated control and display features, is provided as a separate, re-usable unit, that may be used as standard equipment in operating rooms, for example. In the system illustrated, console unit 12 is not contaminated by contact with blood or aspirate during operation, and the power and control systems are durable and long-lasting and may be reused for many interventions. Console unit 12 may be provided in a housing designed to sit on a platform during operation, or the housing may be designed for mounting on a portable structure, such as an i.v. pole or another structure. The interventional catheter system, comprising the catheter system 32 with operating head 40, controller 30, aspirate conduit 22, aspirate collection vessel 24, and infusion conduit 18 may be provided as a sterile, single use system kit.

The catheter system and operating head are described below with reference to a rotatable operating head employing a cutter assembly having a plurality of cutter blades for material removal. In interventional catheter applications incorporating aspiration and/or infusion systems, aspiration and/or infusion conduits terminate at or within controller 30, where they communicate with aspiration and infusion lumens within the catheter system 32. A rotatable drive shaft for driving the operating head is provided in catheter system 32. A guidewire may also transit controller 30 and catheter system 32. In general, controller 30 or an associated control mechanism provides user-operated mechanisms for rotating and/or translating the operating head. Controller 30, which is constructed from a durable, sterilizable material, such as hard plastic, may be provided in any convenient ergonomic design and constructed for placement in proximity to and/or in contact with the external body. In one embodiment, the controller may include an integrated handle for operator convenience in holding and supporting the controller during operation. Catheter system 32, exiting controller 30, is axially translatable with respect to controller 30 as the operating head and catheter system are guided to a target material removal site. It will be appreciated that some of the control and operational features described herein with reference to controller 30 may be provided in console unit 12 and, likewise, some of the control and operational features described with reference to console unit 12 may be provided in controller 30.

The operating head 40 of the interventional catheter disclosed herein may comprise any of a variety of rotational cutting devices or assemblies having one or more cutting surface(s) for cutting, fragmentizing, pulverizing, ablating, scraping, grinding or otherwise reducing the size of undesired material and/or separating undesired material from healthy tissue, such as the walls of a blood vessel, in proximity to the target removal site. In certain embodiments of interventional catheters of the present disclosure, non-rotational operating heads may also be used that incorporate alternative material removal modalities, such as laser or ultrasound ablation techniques, or other types of ablation techniques.

Rotational operating heads having differential cutter assemblies may be provided, as described in the U.S. patent publications cited herein. Operating heads comprising abrasive rotational surfaces may also be used. The operating head, or subcomponents thereof, such as the cutting surfaces, may be coated with a radio-opaque material such as gold, platinum, inks and the like, to render the operating head radioscopically visible and to assist a medical professional in guiding and positioning the cutter assembly relative to an occlusion.

Exemplary materials for construction of the cutting surface(s) of cutter assemblies of the present invention include metals, metal alloys, ceramics and cermet materials such as, but not limited to, various types of stainless steels, such as series 300 and/or 400, vanadium steel, nickel-titanium, titanium, titanium-containing metals and oxide ceramics. Metallic materials such as stainless steels may be hardened using well-known techniques. In general, cutter surfaces are constructed from hard materials and may be treated to impart even greater hardness to the cutter surfaces.

Fig. 2A illustrates a fixed blade cutter assembly having a plurality of differential cutting surfaces according to the present invention. Cutter assembly 40 comprises a plurality of cutter blades 42 arranged in a radially symmetrical arrangement with respect to a central longitudinal axis of the cutter assembly. Each of the cutter blades 42 is joined at a distal end to form a distal bore 44 which serves as a rotating bearing for a guidewire 45. In cutter assemblies that are employed without guidance over a guidewire, cutter blades 42 may alternatively terminate at their distal ends in a blunt or rounded or pointed structure without forming a distal bore. Cutter blades 42 terminate at their proximal ends in a proximal ring-like collar 46.

In various types of interventional catheters, cutter assembly 40 may be mounted to a drive shaft, a catheter system, or an intermediate bearing structure. Fig. 2 illustrates an embodiment in which cutter assembly 40 is mounted to a rotating element 52 of bearing 50. A non-rotating element 54 of the bearing is mounted to a distal portion of catheter 56 or another cylindrical structure provided at a distal portion of the interventional catheter. The rotating bearing element and cutter assembly are rotated during operation by the drive shaft, while the non-rotating element of the bearing structure and the distal portion of the catheter remain stationary during rotation of the operating head.

The overall outer configuration of cutter assembly 40 is generally round from an axial view and oblong or frusto-conical from a profile view. The outer edges of cutter blades 42 taper along a curved line between smaller diameter distal bore 44 and larger diameter proximal collar 46, such that the diameter of the cutter assembly decreases toward its distal end. This configuration allows the smaller diameter distal end of the cutter assembly to penetrate an obstruction or partial obstruction while providing a progressively larger bore as the cutter assembly is advanced through the obstruction.

Each of the cutter blades 42 has a differential cutter surface on a leading edge 43 that contacts material to be removed when cutter assembly 40 is rotated during a material removal operation. Differential cutter surfaces may be optimized for use in different types of cutting environments, for different types of materials being removed, and for different applications, by providing different cant angles. In general, the higher the cant angle the more aggressive the action of the cutter surface. A cutter surface having a cant angle of less than 90° has previously been shown to be gentle and benign when it contacts a resilient surface, such as the wall of a body lumen, yet it effectively cuts and abrades less resilient materials, such as plaque, calcified material and thrombus, to provide effective removal of disease material. Cutter assemblies disclosed herein are benign to healthy, elastic tissue while providing effective removal of less elastic, disease tissue.

The leading edge cutter surfaces are preferably generally smooth, although they may be provided as abrasive surfaces, or surfaces coated with abrasive materials, in alternative embodiments. The use of diamond grit or other types of abrasives on the cutting surfaces may reduce the size of particles generated during rotation of cutter assemblies, thereby improving the efficiency of removal of the particles and reducing the risk of undesirable side effects. The use of diamond grit or other types of abrasives is particularly advantageous when removing hard materials, and/or small, targeted areas of unwanted material. The diamond grit or other type of abrasive employed on the cutting surfaces may have a particle size of about 400 microns or less. In some embodiments, the abrasive materials have a particle size of about 100 microns or less, while in some embodiments, the abrasives have a particle size of about 40 microns or less, generally between about 20 and 40 microns in size.

The cant angle α formed by the leading edges 43 of cutter blades 42 and a line tangent to a circle circumscribing the outer edges of the cutter surfaces are illustrated in Fig. 2B. Cant angles α formed by leading edges 43 of cutter surfaces 42 are preferably less than 90°. In some embodiments of fixed blade cutter surfaces disclosed herein, the cant angle of the cutter blades is less than 80° and, in some embodiments, is about 70°. Trailing angles β formed by trailing edges 45 of cutter surfaces 42 are preferably less than the corresponding cant angles and, in many embodiments, are less than 70°.

The number of cutter blades provided on a fixed cutter assembly of the present invention may vary depending on the interventional application and the nature of the material being removed. As few as three blades may be provided and as many as 12-15 blades may be provided in a radially symmetrical arrangement. Fixed blade cutter assemblies having 7 or more blades are preferred for many applications. In one embodiment, as illustrated in Fig. 2A, a plurality of generally oval ports 48 may be provided between neighboring blades for communication with an internal space of the cutter assembly. Ports 48 may be provided between each pair of neighboring blades, as illustrated, or fewer ports may be provided. In one embodiment, as illustrated, ports 48 are preferably provided in a proximal portion of the cutter assembly in proximity to the proximal collar. Ports 48 may be used as aspiration or infusion ports and communicate with an appropriate aspiration or infusion lumen provided within catheter 56. Additional aspiration and/or infusion ports may be provided in locations proximal to the cutter assembly.

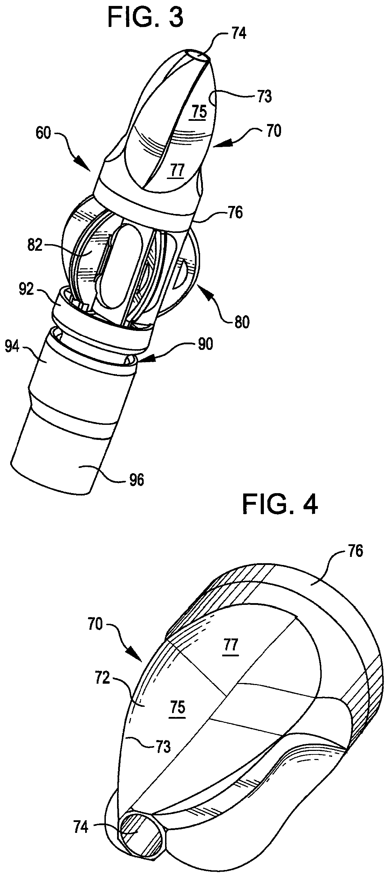

Fig. 3 illustrates another cutter assembly for interventional catheters of the present invention in which a dual cutter assembly 60 comprises a fixed blade cutter 70 and an adjustable blade cutter assembly 80. In various types of interventional catheters, cutter assembly 60 may be mounted to a drive shaft, a catheter system, or an intermediate bearing structure. Fig. 3 illustrates an embodiment in which cutter assembly 60 is mounted to a rotating element 92 of bearing 90. A non-rotating element 94 of the bearing is mounted to a distal portion of catheter 96 or another cylindrical structure provided at a distal portion of the interventional catheter. The rotating bearing element 92 and cutter assembly 60 are rotated during operation by the drive shaft, while the non-rotating element of the bearing structure 94 and the distal portion of the catheter 96 remain stationary during rotation of the operating head.

Fixed blade cutter assembly 70, illustrated in isolation in Fig. 4, comprises a plurality of differential cutting surfaces 72. The overall outer configuration of cutter assembly 70 is generally round from an axial view and oblong or frusto-conical from a profile view. The outer edges of cutter blades 72 taper along a curved line between smaller diameter distal bore 74 and larger diameter proximal collar 76, such that the diameter of the cutter assembly decreases toward its distal end. This configuration allows the smaller diameter distal end of the cutter assembly to penetrate an obstruction or partial obstruction while providing a progressively larger bore as the cutter assembly is advanced through the obstruction.

Each of the cutter blades 72 is fixed on the cutter assembly and preferably has a differential cutter surface on a leading edge 73 that contacts material to be removed when cutter assembly 70 is rotated during a material removal operation. The leading edge cutter surfaces are preferably generally smooth, although they may be provided as abrasive surfaces, or surfaces coated with abrasive materials, in alternative embodiments. The blades may be provided as generally planar surfaces 75 at a distal portion, terminating proximally in a curved or scooped area 77. The cant angles formed as described above by the leading edges 73 of cutter surfaces 72 and a line tangent to a circle circumscribing the outer edges of the cutter surfaces at each cutter surface are preferably less than 90°. In some embodiments of fixed blade cutter surfaces disclosed herein, the cant angle of the cutter blades is less than 80° and, in some embodiments, is about 70°. The trailing angles β formed by the trailing edges 78 of cutter surfaces 72 and a line tangent to a circle circumscribing the edges of the cutter surfaces are generally less than the cant angle α and, in many embodiments, are less than 70°.

The fixed blade cutter assembly 70 does not have ports or openings, other than the guidewire bore 74, communicating with an interior of the assembly. Aspiration and/or infusion ports may be provided elsewhere in proximity to the cutter assembly, if desired. The number of cutter blades provided on fixed cutter assembly 70 of the present invention may vary depending on the interventional application and the nature of the material being removed. As few as three blades may be provided and as many as 5-7 blades may be provided in a radially symmetrical arrangement. Fixed blade cutter assemblies having 3 or more blades are preferred for many applications.

Cutter assembly 60 illustrated in Fig. 3 also incorporates an adjustable blade cutter assembly 80 having a plurality of cutter surfaces 82 presenting a different cutting profile than that of cutter surfaces of the distal blades 72. Many different types of adjustable blade cutter assemblies are known in the art and may be used in combination with a fixed blade distal cutter assembly of the present invention. Cutter assembly 80 may comprise a plurality of pivoting blades, for example, that move between a tangential, non-cutting, position and a radial, cutting, position by changing the direction of rotation of the cutter assembly. The fixed blade cutter assembly may be operated in one direction of rotation in a first cutting operation to clear a passageway, and the adjustable blade cutter may be operated in an opposite direction of rotation in a second cutting operation to clear a larger opening in a lumen or cavity. Cutter assemblies of this type are described in the patent publications incorporated herein by reference.

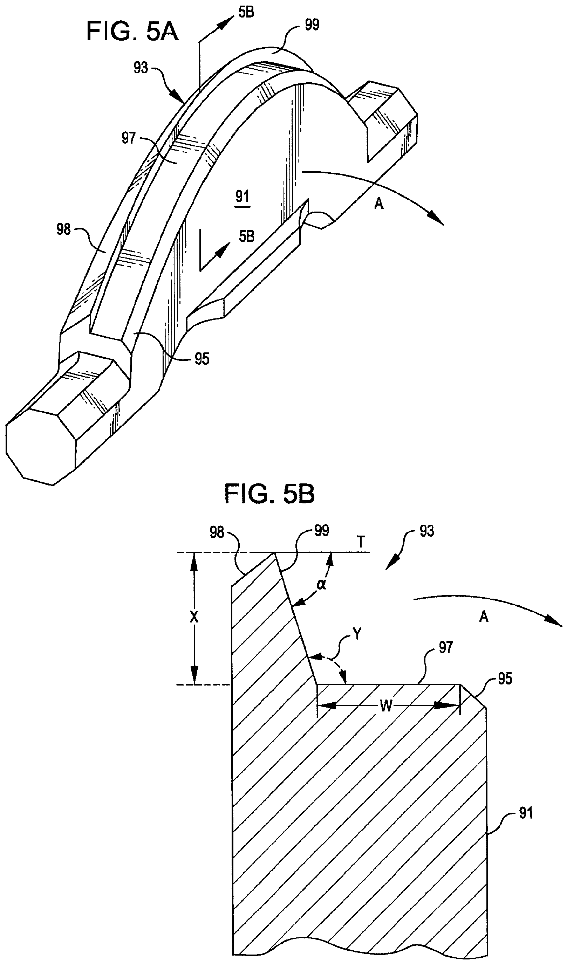

Figs. 5A and 5B illustrate a stepped cutter blade configuration that may be incorporated in cutter assemblies of the present invention. While cutter blade 93 is illustrated as a generally fan-shaped, flat blade suitable for use, for example, in an adjustable blade cutter assembly, it will be appreciated that the stepped blade cutter configuration may be incorporated in cutter blades having a variety of configurations, including fixed cutter blades and different configurations of adjustable cutter blades. Cutter blade 93 illustrated in Figs. 5A and 5B is designed to rotate in a clockwise direction A. Cutter blade 93 has a leading edge cutting surface 99 adjacent or in proximity to a shoulder region 97 and a trailing surface 98. An angled or chamfered surface 95 may be provided between shoulder region 97 and the leading surface 91 of the cutter blade. As cutter blade 93 rotates in clockwise direction A, the leading surface 91 or chamfered edge 95 of blade 93 contacts the tissue first, followed by the shoulder 97 and cutter surface 99. The leading surface 91, chamfered edge 95 and shoulder 97 are generally atraumatic to tissue, while cutter surface 99 exerts a material removal action. Cutter surface 99 preferably forms a cant angle α of from about 60° to about 110°, more preferably from about 70° to about 100°, and more preferably yet from about 80° to about 95°, with respect to a line T tangent to the circumscribed arc of the circumference of the cutter assembly.

The height "X" of the cutter surface 99 from its outer edge to the point where it joins shoulder 97 is one factor that governs the "aggressiveness" of the cutter blade. For a given cant angle α, a shorter cutter surface X removes generally less material per pass than a longer cutter surface X. For many atherectomy and thrombectomy applications, cutter surface X may have a height X of from about 0.0127 to 0.254 mm (about 0.0005 to 0.010 inch), with heights of from about 0.025 to 0.076 mm (0.001 to 0.003 inch) being especially preferred. The height X may be substantially uniform along the length of the cutter surface, or it may taper along the length of the cutter assembly. In general, the cutter surfaces provided on a cutter assembly have the same or a similar cant angle and height X, but when multiple cutter assemblies are provided, the cant angles and heights X of cutter blades provided on the different cutter assemblies may be varied to vary the cutting properties of the assemblies.

Shoulder region 97 is provided adjacent or in close proximity to cutter surface 99 and may have a substantially flat profile, or an angled or curved profile. The angle "Y" formed by the plane of cutter surface 99 and shoulder 97 is preferably from about 45° to about 150°, more preferably from about 60° to about 120°, and more preferably yet from about 80° to about 110°. The width "W" of shoulder region 97 is preferably greater than the height X of cutter surfaces 99. In one embodiment, the width W of shoulder region 97 is from about 1.5 to about 10.0 times the height X of cutter surface 99; in another embodiment the width W of shoulder region 97 is from about 2.0 to about 4.0 times the height X of cutter surface 99. For atherectomy and thrombectomy applications, the width W of shoulder 97 is preferably from about 0.025 to about 0.381 mm (about 0.001 to about 0.015 inch), and the width W of shoulder 97 is more preferably from about 0.076 to about 0.254 mm (0.003 to about 0.010 inch).

Optional chamfered surface 95 may be provided as an angled or curved surface between the leading blade surface 91 and shoulder 97. Chamfered surface 95 is generally narrower than shoulder 97 and may have a constant width over its length, or it may taper from one end to another, or toward and away from a central blade portion. Chamfered surface 95 and shoulder 97 contact and displace the surface of tissue prior to contact between the tissue surface and the cutting surface 99, thereby restricting the depth of the "bite" that cutter surface 99 takes in the tissue and reducing the risk of damage to tissue. The protective effect of chamfered surface 95 and shoulder 97 enables the use of cutting surfaces having more aggressive cant angles, for example cant angles of about or greater than 80°, in order to effectively cut hardened disease material, while providing protection to healthy elastic tissue.

Cutter blades 93 may additionally be provided with cut-outs or cavities that penetrate their surfaces in locations that are radially inward of the cutter surfaces and shoulders. In one embodiment, a curved slot having a curve that substantially matches the outer edge of the cutter surface may be provided, for example, to reduce drag produced by rotation of the blade against fluids. In another embodiment, multiple slots or openings may be provided through the surface of cutter blades 93. In general, the slots or openings represent at least about 5% of the surface area of the blade surface and more typically represent at least about 10% to 15% of the surface area of the blade surface. Slots or openings provided in the cutter blade surfaces generally represent less than about 50% of the blade surface area.

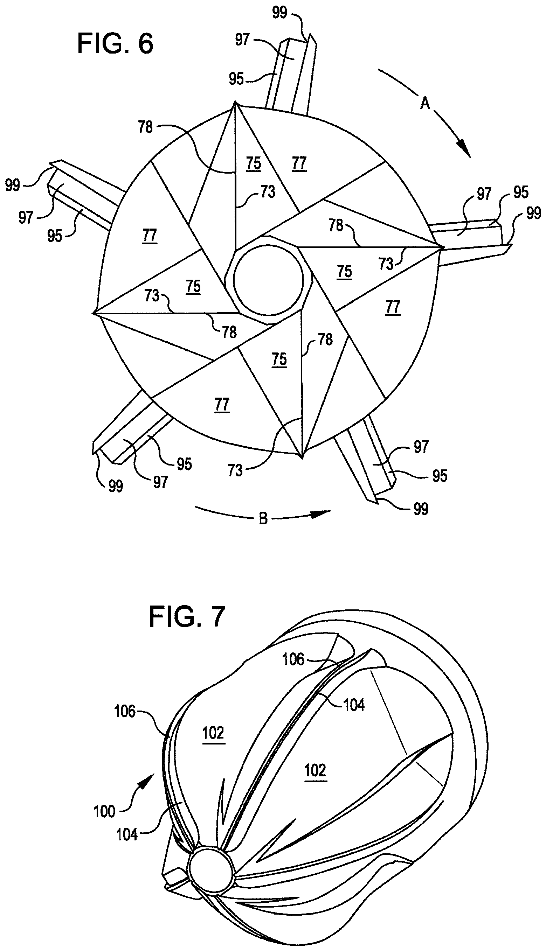

Fig. 6 illustrates an end view of an operating head of the type illustrated in Fig. 3 having a distal cutter assembly of the type illustrated in Fig. 4 and a proximal cutter assembly having stepped blades of the type illustrated in Figs. 5A and 5B . The fixed and adjustable blade cutter assemblies provide different cutting profiles and operate in opposite directions of rotation of the operating head. The distal fixed cutter 70 operates as the primary cutter assembly when the operating head is rotated in clockwise direction A. Leading blade surfaces 73 provided at the edges of leading blade faces 75 do not have a stepped configuration and preferably have cant angles α of less than about 80°, generally from about 65° to about 90°. Trailing blade surfaces 78 preferably have cant angles of less than that of the leading blade faces. Proximal cutter assembly surfaces 77 preferably have a curved or scooped configuration, which is not apparent from the figure.

Proximal blades 93 have a stepped cutter surface configuration and operate as the primary cutters when operating head 60 is rotated in counterclockwise direction B. Stepped cutter blades 93 present a cutter surface 99 adjacent shoulder 97, and chamfered edge 95 is presented as a leading surface. When the operating head of Fig. 6 is rotated in counterclockwise direction B, tissue is contacted first by chamfered edge 95 (and, to some degree, the leading blade surface positioned radially inwardly from chamfered edge 95) and shoulder 97, which present substantially atraumatic surfaces, and stepped cutter surfaces 99 operate as the primary cutter surfaces. Cutter blades 93 preferably assume a general tangential orientation when the operating head is rotated in clockwise direction A.

Stepped cutter blades of the present invention may be provided on a variety of fixed and adjustable cutter blades, and on fixed and adjustable cutter assemblies. An enlarged view of a fixed blade cutter assembly similar to that described with reference to the cutter assembly of Fig. 4 is shown in Fig. 7. In this embodiment, cutter assembly 100 has a plurality of cutter blades 102 oriented for operation by rotation in a counterclockwise direction B. Cutter blades 102 incorporate upstanding cutter surfaces 106 adjacent shoulders 104. The embodiment illustrated in Fig. 7 does not incorporate a chamfered edge, but the shoulders 104 provide generally atraumatic contact with tissue. The trailing edges of cutter blades 102 may be provided at a shallow angle to cutter surfaces 106, providing a relatively blunt circumferential edge on cutter blades 102.

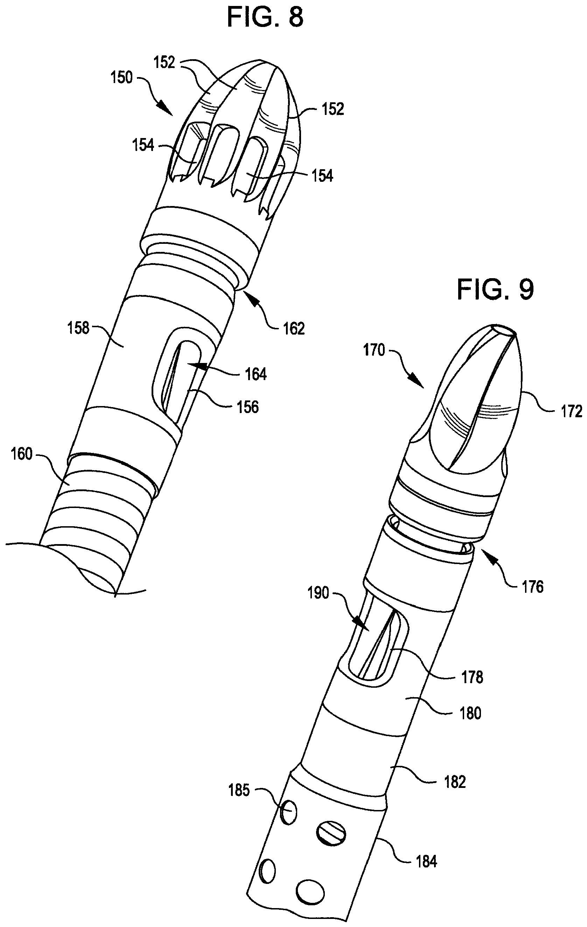

Fig. 8 illustrates the distal end of one embodiment of an interventional catheter of the present invention. In this embodiment, the operating head comprises a multi-bladed cutter assembly 150 having a plurality of raised blades 152 arranged in a radially symmetrical configuration. Blades 152 are preferably differential cutting blades, and cutting assembly 150 may incorporate a plurality of ports 154 arranged in a radially symmetrical configuration in the spaces between blades 152. Ports 154 are shown provided between each set of neighboring blade structures in Fig. 8, but it will be appreciated that fewer ports may be provided. Ports 154 are preferably provided in a generally proximal portion of cutter assembly 150 and may have a generally oblong configuration, as illustrated, or may take a variety of other configurations.

The distal end of the interventional catheter illustrated in Fig. 8 additionally comprises a large port 156 located in a distal portion of the catheter, or a proximal portion of the cutter assembly, proximal to blades 152. Port 156 is generally provided as a window or cut-out in a cylindrical structure and preferably spans at least 10% of the circumference of the structure; more preferably at least 20% of the circumference of the structure; and yet more preferably at least 30% of the circumference of the structure. The cylindrical structure supporting port 156 may be a distal catheter portion, or port 156 may be provided in a generally cylindrical tubular shell structure mounted, directly or indirectly, to a distal catheter portion 160. In one embodiment, illustrated in Fig. 8, a rigid cylindrical shell 158 is mounted to distal catheter portion 160 at its proximal end and is mounted to, or forms a stationary element of, bearing 162 at its distal end. Bearing 162 allows distal catheter portion 160 and cylindrical shell 158 to remain stationary during rotation of cutting assembly 150. Bearing 162 may also provide limited articulation of cutting assembly 150 about its longitudinal axis.

Ports 154 may be operated as aspiration or infusion ports and, likewise, enlarged proximal port 156 may be operated as an aspiration or infusion port. In one embodiment, proximal port 156 is provided as an aspiration port and communicates with an aspiration lumen within catheter 160 that communicates with aspiration conduit 22, while ports 154 operate as infusion ports and communicate with an infusion lumen within catheter 160 that communicates with infusion conduit 26. In another embodiment, port 156 is provided as an infusion port and communicates with an infusion lumen within catheter 160 that communicates with infusion conduit 26, while ports 154 operate as aspiration ports and communicate with an aspiration lumen within catheter 160 that communicates with aspiration infusion conduit 22. In another embodiment, ports 154 may be operated as aspiration or infusion ports and enlarged proximal port 156 operates as an aspiration port and incorporates a rotating macerator assembly 164, which is described in greater detail below.

In the embodiment illustrated in Fig. 9, operating head 170 comprises a distal cutter assembly 172 mounted to a bearing system 176 that allows rotation of the operating head while the catheter components proximal to the operating head remain stationary during rotation. The interventional catheter illustrated in Fig. 9 additionally comprises a proximal aspiration port 178 provided as an opening in a cylindrical shell structure 180 located proximal to the cutter assembly 172. Aspiration port 178 communicates with an aspiration lumen within catheter 182 that communicates proximally with aspiration conduit 22.

Proximal aspiration port 178 may be provided as an opening or window in a distal catheter portion, or it may be provided as an opening in a substantially rigid cylindrical shell 180, as illustrated. Cylindrical shell 180 is constructed from a generally rigid, durable material, such as surgical steel or stainless steel, and has a length that is approximately the same as that of the cutter assembly. Shell 180 is generally mounted to, or forms a stationary component of, bearing 176 at its distal end. Bearing 176 allows catheter 182 and cylindrical casing 180 to remain stationary during rotation of operating head 150. Bearing 176 may also provide limited articulation of operating head 170 about its longitudinal axis.

Aspiration port 178 may be provided as a window spanning at least 15% of the circumference of the shell structure; more preferably at least 25% of the circumference of the shell structure; and yet more preferably at least 35% of the circumference of the shell structure. The proximal aspiration port may be provided, for example, in a generally ovoid, rectangular, or square profile. The surface area, or size of the opening of port 178 depends on the relative sizes of the operating head, the catheter and the size of debris that is anticipated will be generated during operation of the operating head. In one embodiment, port 178 has a length in the direction of the longitudinal axis of the interventional catheter that is from about 0.5 to about 2.5 times the transverse width of the port as measured on the arc of the cylindrical surface, preferably from about 0.7 to about 2.0 times the transverse width of the port. In certain embodiments, aspiration port 178 has an opening, or surface area that is between about 0.5 to about 20 mm², preferably between about 0.5 and 10 mm² in surface area.

In one embodiment, the interventional catheter of Fig. 9 incorporates a macerator assembly 190 mounted for rotation inside cylindrical shell 180. Macerator assembly 190 rotates within the inner cavity of cylindrical shell 180 and has projecting bars, described in detail below, that interact with walls of the aspiration port 178 and the inner surface of cylindrical shell 180 to grind or macerate debris that is aspirated through port 178.

The interventional catheter illustrated in Fig. 9 additionally incorporates a plurality of infusion ports 185 located proximally with respect to aspiration port 178, but in proximity to the aspiration port and operating head 170. Infusion ports 185, which may be provided in an outer sheath 184, as shown in Fig. 9 , communicate with an infusion lumen in the catheter assembly, which communicates with infusion conduit 26. In certain embodiments, between two and twenty, for example twelve, infusion ports 185 are provided in an infusion sheath 184 mounted to distal catheter 182. The infusion ports may have a generally uniform size, or infusion ports of different sizes may be provided. The infusion ports may be generally cylindrical, as shown in Fig. 9 , or they may have alternative configurations. Each infusion port 185 may have a diameter of approximately 0.127 to 5.1 mm (approximately 0.005 in. to 0.20 in.), more preferably from about 0.152 to about 0.381 mm (about 0.006 in. to about 0.015 in.). Infusion ports having diameters of about 0.254 mm (about 0.010 in.) are especially preferred for certain applications. In one embodiment, the infusion ports are spaced in a generally circumferential pattern to provide a substantially uniform flow of infusate around the circumference of the infusion sheath.

In an alternative embodiment, a single infusion port having a surface area generally equal to or less than the surface area of the aspiration port may be provided proximally to the aspiration port. A plurality of infusion ports may also be spaced relatively closely and arranged to provide targeted infusion, resulting in a higher volume or pressure of infusate at different locations around the circumference of the infusion sheath. Providing a large infusion port, or a series of infusion ports in close proximity to one another allows directional flow of infusate and may facilitate aspiration of debris and/or positioning of the operating head.

In certain embodiments, the infusion rate and pressure is relatively low, unlike many prior art systems in which a high rate of fluid infusion is employed to break-up unwanted material or to guide an operating head. In many embodiments, for example, the pressure of the infusion fluid as measured at the infusion pump may from about 0.55 to 1.38 MPa (80 to 200 psi). The volume ratio of infusate to aspirate is also important in many applications. In systems of the present invention, the rate of infusion is generally greater than the rate of aspiration. The volume ratio of infusate to aspirate is greater than about 1:1 and less than about 2.5:1. For some applications, the volume ratio of infusate to aspirate may be greater than about 1.5:1 and is less than about 2.5:1. In some embodiments, the volume ratio of infusate to aspirate is approximately 1.5:1, 2:1 or 2.5:1. In certain embodiments, the rate of infusion is approximately 45-100 ml/min of device run-time, and the rate of aspiration is approximately 20-60 ml/min of device run-time. In certain embodiments, the rate of infusion is approximately 45-150 ml/min, for example 50-90 ml/min, of device run-time, and the rate of aspiration is approximately 20-90 ml/min, for example, 20-60 ml/min of device run-time.

Various control and feedback mechanisms may be used in connection with the aspiration and infusion systems in interventional catheters of the present invention. In one embodiment, for example, a bubble detection device is provided in proximity to an infusate conduit and operates to detect gas bubbles in the infusate fluid. An alarm may be triggered upon detection of a bubble, or a control mechanism may inactivate the infusion and aspiration systems in response to detection of a bubble. Power to the operating head may also be inactivated upon detection of a bubble to inactivate the system and prevent introduction of the bubble into the patient.

Activation and inactivation of the infusion and aspiration systems may be controlled by an operator using operator selectable features and controls. Alternatively, various operating protocols may be programmed into the system or provided as selectable features. Operation of the aspiration and infusion systems may be coordinated in a variety of ways, for example, with operation of the operating head. Activation and inactivation of the operating head is generally controlled by the operator. In one embodiment, the infusion system is primed to ensure that the infusate conduit is filled with liquid and a reliable supply of infusate to infusion ports in proximity to the operating head is established prior to guidance of the interventional catheter to the site of intervention. In one embodiment, the infusate and aspiration systems are activated automatically upon activation of the operating head. In another embodiment, the infusate and aspiration systems are activated after a generally short delay period following activation of the operating head. The infusion and aspiration systems may also be inactivated automatically upon inactivation of the operating head. Alternatively, the infusion and/or aspiration systems may be inactivated after a predetermined or selectable time period following inactivation of the operating head. The system may optionally incorporate override controls that allow an operator to override a programmed infusion or aspiration feature.

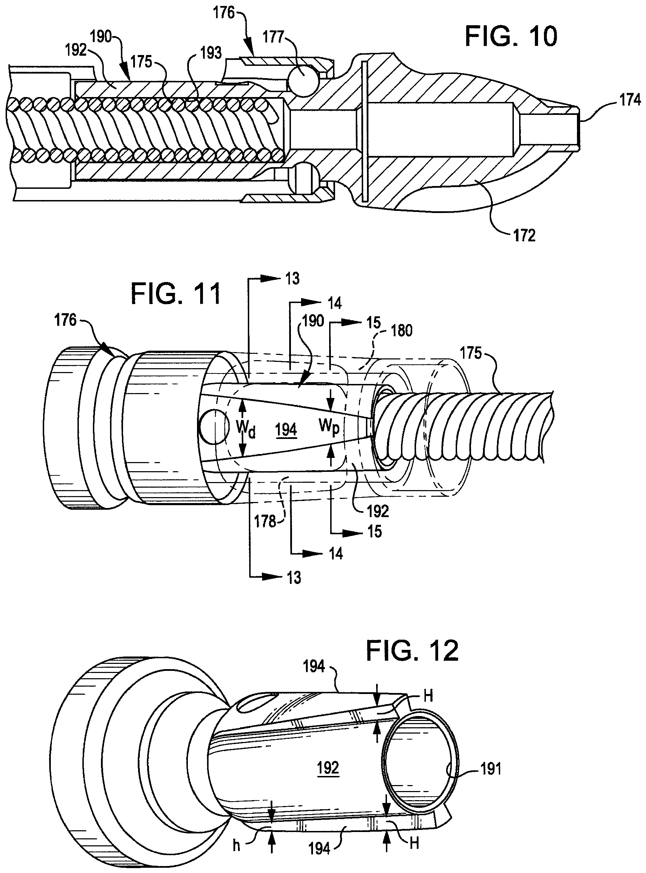

Fig. 10 shows a cross-sectional view of the distal end of the interventional catheter illustrated in Fig. 9. Distal cutter assembly 172 has an internal guidewire lumen terminating in guidewire bore 174. This view illustrates a helical rotational drive 175, which is mounted inside a central cavity in the core structure of macerator assembly 190 and fixed to the macerator, providing rotation of macerator assembly 190. In embodiments employing a rotating operating head, drive 175 additionally drives the operating head, directly or indirectly. A bearing member 176 comprising a plurality of ball bearings 177 may be provided between the rotating cutter assembly 172 and/or the rotational macerator assembly 190 and the static cylindrical casing 180 and catheter 182.

The rotating macerator 190, as illustrated in Figs. 11 and 12, comprises a central core structure 192 and at least one projecting bar 194 aligned generally with the longitudinal axis of the interventional catheter. The central core structure 192 preferably has a generally cylindrical internal bore 191. Multiple projecting bars 194 may be provided and are preferably arranged in a radially symmetrical arrangement with respect to the longitudinal axis of the catheter assembly. In one embodiment, illustrated in Figs. 12-15, two projecting bars are provided.

Projecting bars 194 preferably have a curved exterior surface that generally matches and rotates freely in the interior surface of cylindrical casing 180. The width W of the arced exterior surface of the upstanding bars 194 tapers from a distal end (W_{d}) to a proximal end (Wₚ) of the rotating macerator. The proximal portion of projecting bar 194 has a narrower profile Wₚ that may be from about 5% to about 75% the width of the distal end profile W_{d}. In one embodiment, the external diameter of central core structure 192 tapers from a larger diameter at the distal end to a smaller diameter at the proximal end. Consequently, upstanding bars 194, as illustrated, may have a higher profile with respect to the surface of the central core structure 192 at the proximal end than at the distal end. In some embodiments, the radial height H of projecting bars 194 at a proximal portion measured from the surface of central core structure 192 is preferably at least about 50% greater than the radial height h of projecting bars 194 at a distal portion of the macerator assembly measured from the surface of central core structure 192. In some embodiments, the radial height H of projecting bars 194 at a proximal portion measured from the surface of central core structure 192 is preferably at least about 100% greater than the radial height h of projecting bars 194 at a distal portion of the macerator assembly.

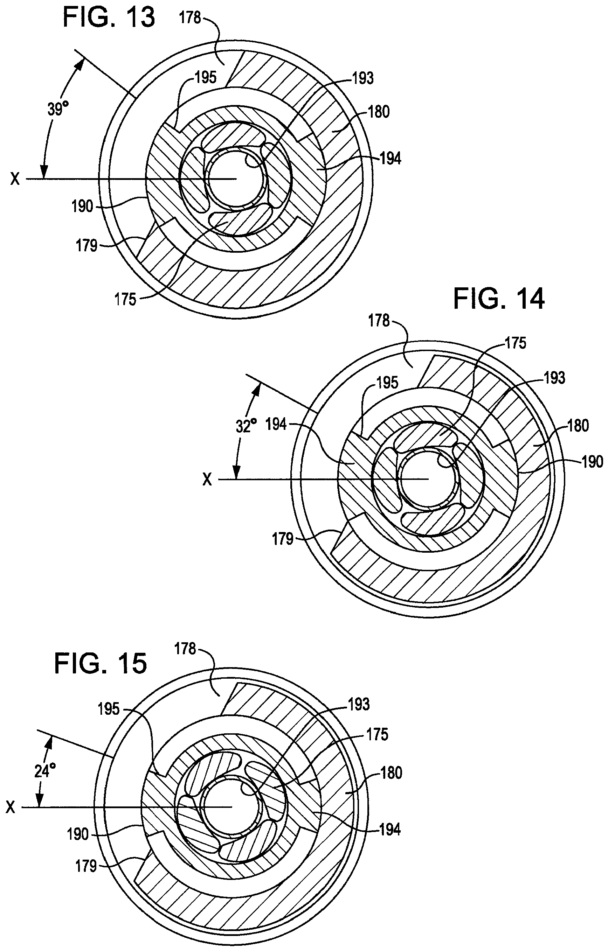

Figs. 13-15 illustrate the geometries of the macerator element and upstanding bars at various points along the macerator assembly 90, with Fig. 13 illustrating a cross-sectional view at a generally distal end of the macerator assembly, Fig. 14 illustrating a cross-sectional view at a generally central portion of the macerator element, and Fig. 15 illustrating a cross-sectional view at a generally proximal end of the macerator element. A multi-filar drive 175 and a central guidewire lumen 185 are visible. Window 178 is shown as a cut-out section of cylindrical casing 180 having a generally blunt perimeter wall 179. The outer walls 193 of projecting bars 194 are illustrated having an arced surface that generally matches the internal surface of cylindrical casing 180 so that macerator assembly 190 is freely rotatable within cylindrical casing 180 about the longitudinal axis of the interventional catheter. The change in width of upstanding bars 194 from a wider distal end illustrated in Fig. 13 to a narrower proximal end illustrated in Fig. 15 is also evident.

Angled walls 195 are provided between the outer walls 193 of projecting bars 194 and the exterior surface of central core structure 192. Angled walls 195 are generally configured to present an undercut angle of from about 10° to about 60° with respect to a line bisecting the upstanding bar and intersecting the central longitudinal axis of the macerator assembly. The undercut angles of angled walls 195 may be from about 20° to about 45° in certain embodiments. The undercut angle of walls 195 is preferably greater at the distal end of the macerator assembly, as illustrated in Fig. 13, and less at the proximal end of the macerator assembly. In the embodiment illustrated, the undercut angle is 39° at a distal portion of the macerator device (Fig. 13); 32° at a central portion of the macerator device (Fig. 14) and 24° at a proximal portion of the macerator device (Fig. 15). The difference in undercut angle between a distal end and a proximal end of the macerator assembly is preferably at least about 10°, more preferably at least about 15° and, in some embodiments, up to about 20° or 30°.

The angle formed between the undercut angle of walls 195 of the projecting bar(s) and the face 179 of window 178 may also be adjusted to facilitate breaking down of particulates. In some embodiments, as illustrated in Fig. 14, the angle formed by the undercut angle of the projecting bar wall and the face of the aspiration window is about 32°. This angle also changes with changes in the undercut angles of the projecting bars and may range from about 15° to about 60° from the distal to the proximal end of the macerator assembly, more preferably from about 20° to about 50°.

During operation of the cutter assembly or operating head, the central core and upstanding bars of macerator assembly 190 rotate, while the aspiration port and cylindrical shell structure in which the aspiration port is provided remain static. As the upstanding bars rotate and debris is aspirated through the aspiration window, the upstanding bars interact with the wall of the aspiration port and the interior surface of the cylindrical structure to shear, grind, macerate and/or break-up debris as it enters the aspiration port and is aspirated through the aspiration lumen. The tapered structure and undercut walls of the upstanding bars also facilitate movement of fluid and debris proximally in the macerator assembly to the aspiration lumen. The rotating element of the macerator assembly does not extend radially beyond the inner wall of the cylindrical structure and aspiration port, and is generally constructed from a rigid material such as, but not limited to, hardened stainless steel, titanium or titanium nitrate-coated stainless steel.

The present invention has been described with reference to specific embodiments and figures. These specific embodiments should not be construed as limitations on the scope of the invention, but merely as illustrations of exemplary embodiments. It is further understood that many modifications, additions and substitutions may be made to the described interventional catheter and control system without departing from the scope of the present invention which is defined solely by the claims.

## Claims

1. An interventional catheter assembly (10) comprising:
an operating head (40; 60; 100; 150; 170) mounted at a distal end of the catheter assembly and comprising a system for removing obstructive material from a target site in a body lumen or a cavity;
at least one aspiration port (156; 178) provided in proximity to the operating head and in communication with a sealed lumen for withdrawing aspirate fluids and obstructive material from the target site;
at least one infusion port (185) for supplying infusate fluid to a location in proximity to the target site;
an aspiration system (16, 22) for applying vacuum to the at least one aspiration port (156; 178); and
an infusion system (14, 18, 20) for providing infusate fluid to the at least one infusion port (185), wherein the infusion and aspiration systems are controlled during operation of the interventional catheter assembly (10) to provide volume ratios of infusate to aspirate of greater than about 1:1 and less than about 2.5:1;
wherein the at least one aspiration port (156; 178) is located proximal to the operating head (150; 170);
wherein the at least one infusion port (185) is located proximal to the at least one aspiration port (178).

2. The interventional catheter assembly of claim 1, wherein the volume ratios of infusate to aspirate is greater than about 1.5:1 and less than about 2.5:1.

3. The interventional catheter assembly of any one of the preceding claims, wherein the operating head comprises a cutter assembly (40; 60; 100; 150; 172) having a plurality of cutter blades (42; 93; 102; 152).

4. The interventional catheter assembly of claim 3, wherein the cutter assembly (60) comprises a fixed blade cutter assembly (70) and an adjustable blade cutter assembly (80).

5. The interventional catheter assembly of claim 4, wherein the adjustable blade cutter assembly (80) has a different cutting profile than the fixed blade cutter assembly (70).

6. The interventional catheter assembly of claim 5, wherein the adjustable blade cutter assembly (80) includes a plurality of pivoting blades that move between a non-cutting position and a cutting position by changing a direction of rotation of the cutter assembly.

7. The interventional catheter assembly of any one of the preceding claims, wherein the at least one aspiration port (156; 178) is provided as an opening in a substantially rigid cylindrical shell (158; 180).

8. The interventional catheter assembly of claim 7, further comprising a macerator assembly (164; 190) mounted for rotation inside the cylindrical shell (158; 180).

9. The interventional catheter assembly of claim 8, wherein the macerator assembly (190) has projecting bars (194).

10. The interventional catheter assembly of claim 9, wherein the macerator assembly (164; 190) does not extend radially beyond an inner wall of the cylindrical shell (158; 180).

11. The interventional catheter assembly of any one of the preceding claims, wherein the infusion and aspiration systems are controlled during operation of the interventional catheter assembly (10) to provide an infusion rate of about 45-150 ml/min of device run-time and an aspiration rate of about 20-90 ml/min of device run time.

12. The interventional catheter assembly of any one of the preceding claims, wherein the infusion and aspiration systems are automatically inactivated after a selected time period following inactivation of the operating head (40; 60; 100; 150; 170).

## Patentansprüche

1. Interventionskatheteranordnung (10), die aufweist:
einen Arbeitskopf (40; 60; 100; 150; 170), der an einem distalen Ende der Katheteranordnung installiert ist und ein System zum Entfernen von Obstruktionsmaterial von einer Zielstelle in einem Körperlumen oder einer Körperhöhle aufweist;
mindestens einen Aspirationsport (156; 178), der in der Nähe des Arbeitskopfs und in Kommunikation mit einem abgedichteten Lumen zum Abziehen von Aspiratfluiden und Obstruktionsmaterial von der Zielstelle vorgesehen ist;
mindestens einen Infusionsport (185) zum Zuführen von Infusatfluid zu einem Ort in der Nähe der Zielstelle;
ein Aspirationssystem (16, 22) zum Anlegen eines Vakuums am mindestens einen Aspirationsport (156; 178); und
ein Infusionssystem (14, 18, 20) zum Führen von Infusatfluid zum mindestens einen Infusionsport (185), wobei das Infusionssystem und das Aspirationssystem während des Betriebs der Interventionskatheteranordnung (10) so gesteuert werden, dass sie Volumenverhältnisse von Infusat zu Aspirat über etwa 1:1 und unter etwa 2,5:1 bereitstellen;
wobei der mindestens eine Aspirationsport (156; 178) proximal zum Arbeitskopf (150; 170) liegt;
wobei der mindestens eine Infusionsport (185) proximal zum mindestens einen Aspirationsport (178) liegt.

2. Interventionskatheteranordnung nach Anspruch 1, wobei die Volumenverhältnisse von Infusat zu Aspirat über etwa 1,5:1 und unter etwa 2,5:1 liegen.

3. Interventionskatheteranordnung nach einem der vorstehenden Ansprüche, wobei der Arbeitskopf eine Schneidanordnung (40; 60; 100; 150; 172) mit mehreren Schneidklingen (42; 93; 102; 152) aufweist.

4. Interventionskatheteranordnung nach Anspruch 3, wobei die Schneidanordnung (60) eine Schneidanordnung (70) mit festen Klingen und eine Schneidanordnung (80) mit einstellbaren Klingen aufweist.

5. Interventionskatheteranordnung nach Anspruch 4, wobei die Schneidanordnung (80) mit einstellbaren Klingen ein anderes Schneidprofil als die Schneidanordnung (70) mit festen Klingen hat.

6. Interventionskatheteranordnung nach Anspruch 5, wobei die Schneidanordnung (80) mit einstellbaren Klingen mehrere Schwenkklingen aufweist, die sich durch Ändern einer Drehrichtung der Schneidanordnung zwischen einer Nichtschneidposition und einer Schneidposition bewegen.

7. Interventionskatheteranordnung nach einem der vorstehenden Ansprüche, wobei der mindestens eine Aspirationsport (156; 178) als Öffnung in einer im Wesentlichen starren Zylinderhülle (158; 180) vorgesehen ist.

8. Interventionskatheteranordnung nach Anspruch 7, ferner mit einer Mazeratoranordnung (164; 190), die zur Drehung innerhalb der Zylinderhülle (158; 180) installiert ist.

9. Interventionskatheteranordnung nach Anspruch 8, wobei die Mazeratoranordnung (190) vorstehende Riegel (194) hat.

10. Interventionskatheteranordnung nach Anspruch 9, wobei sich die Mazeratoranordnung (164; 190) nicht über eine Innenwand der Zylinderhülle (158; 180) hinaus radial erstreckt.

11. Interventionskatheteranordnung nach einem der vorstehenden Ansprüche, wobei das Infusions- und Aspirationssystem während des Betriebs der Interventionskatheteranordnung (10) so gesteuert werden, dass sie eine Infusionsrate von etwa 45 bis 150 ml/min Vorrichtungslaufzeit und eine Aspirationsrate von etwa 20 bis 90 ml/min Vorrichtungslaufzeit bereitstellen.

12. Interventionskatheteranordnung nach einem der vorstehenden Ansprüche, wobei das Infusions- und Aspirationssystem nach einer ausgewählten Zeitspanne im Anschluss an eine Deaktivierung des Arbeitskopfs (40; 60; 100; 150; 170) automatisch deaktiviert werden.

## Revendications

1. Ensemble de cathéter chirurgical (10) comprenant :
une tête de commande (40 ; 60 ; 100 ; 150 ; 170) montée sur une extrémité distale de l'ensemble de cathéter et comprenant un système pour retirer une matière d'obstruction d'un site cible dans une lumière ou cavité corporelle ;
au moins un orifice d'aspiration (156 ; 178) positionné à proximité de la tête de commande et en communication avec une lumière étanche pour retirer des fluides aspirés et la matière d'obstruction du site cible ;
au moins un orifice de perfusion (185) pour introduire du fluide perfusé à un endroit à proximité du site cible ;
un système d'aspiration (16, 22) pour appliquer un vide au au moins un orifice d'aspiration (156 ; 178) ; et
un système de perfusion (14, 18, 20) pour fournir du fluide perfusé au au moins un orifice de perfusion (185), dans lequel les systèmes de perfusion et aspiration sont contrôlés pendant le fonctionnement de l'ensemble de cathéter chirurgical (10) pour fournir des rapports de volume de fluide perfusé à fluide aspiré supérieurs à environ 1:1 et inférieurs à environ 2,5:1 ;
dans lequel le au moins un orifice d'aspiration (156 ; 178) est disposé à proximité de la tête de commande (150 ; 170) ;
dans lequel le au moins un orifice de perfusion (185) est disposé à proximité du au moins un orifice d'aspiration (178).

2. Ensemble de cathéter chirurgical selon la revendication 1, dans lequel les rapports de volume de fluide perfusé sur fluide aspiré sont supérieurs à environ 1,5:1 et inférieurs à environ 2,5:1.

3. Ensemble de cathéter chirurgical selon l'une quelconque des revendications précédentes, dans lequel la tête de commande comprend un ensemble de dispositif de coupe (40 ; 60 ; 100 ; 150 ; 172) présentant plusieurs lames de coupe (42 ; 93 ; 102 ; 152).

4. Ensemble de cathéter chirurgical selon la revendication 3, dans lequel l'ensemble de dispositif de coupe (60) comprend un ensemble de dispositif de coupe à lame fixe (70) et un ensemble de dispositif de coupe à lame ajustable (80).

5. Ensemble de cathéter chirurgical selon la revendication 4, dans lequel l'ensemble de dispositif de coupe à lame ajustable (80) présente un profil de coupe différent de l'ensemble de dispositif de coupe à lame fixe (70).

6. Ensemble de cathéter chirurgical selon la revendication 5, dans lequel l'ensemble de dispositif de coupe à lame ajustable (80) inclut plusieurs lames pivotantes qui se déplacent entre une position de non-coupe et une position de coupe en modifiant une direction de rotation de l'ensemble de dispositif de coupe.

7. Ensemble de cathéter chirurgical selon l'une quelconque des revendications précédentes, dans lequel le au moins un orifice d'aspiration (156 ; 178) est fourni comme une ouverture dans une enveloppe cylindrique substantiellement rigide (158 ; 180).

8. Ensemble de cathéter chirurgical selon la revendication 7, comprenant de plus un ensemble de broyeur (164 ; 190) monté pour une rotation à l'intérieur de l'enveloppe cylindrique (158 ; 180).

9. Ensemble de cathéter chirurgical selon la revendication 8, dans lequel l'ensemble de broyeur (190) présente des barres en saillie (194).

10. Ensemble de cathéter chirurgical selon la revendication 9, dans lequel l'ensemble de broyeur (164 ; 190) ne s'étend pas radialement au-delà d'une paroi interne de l'enveloppe cylindrique (158 ; 180).

11. Ensemble de cathéter chirurgical selon l'une quelconque des revendications précédentes, dans lequel les systèmes de perfusion et d'aspiration sont commandés pendant le fonctionnement de l'ensemble de cathéter chirurgical (10) pour fournir un rapport de perfusion d'environ 45-150 ml/min du temps de fonctionnement du dispositif et une vitesse d'aspiration d'environ 20-90 ml/min du temps de fonctionnement du dispositif.

12. Ensemble de cathéter chirurgical selon l'une quelconque des revendications précédentes, dans lequel les systèmes de perfusion et d'aspiration sont automatiquement inactivés après une période de temps choisie suivant l'inactivation de la tête de commande (40 ; 60 ; 100 ; 150 ; 170).
